# EUROPEAN PATENT APPLICATION

(11) **EP 4 309 577 A1**
(43) Date of publication of application: **24.01.2024**
(21) Application number: 22186282.4
(22) Date of filing: 21.07.2022
(51) Int. Cl.: A61B 5/107, A61B 5/00

(54) **SYSTEM AND METHOD OF DERIVING INFORMATION RELATING TO A CAVITY IN BLOOD PERFUSED TISSUE**

(71) Applicant: Sonion Nederland B.V., 2132 LS Hoofddorp (NL)
(72) Inventor: Fransen, Alwin, 2132 LS Hoofddorp (NL); Post, Peter Christiaan, 2132 LS Hoofddorp (NL)
(74) Representative: Inspicos P/S

(57) **Abstract**

It is described a system for deriving information relating to a cavity in blood perfused tissue comprising a cavity having an inner surface,such as ana ear canal, the system comprising a sensor comprising a housing and, in or at the housing, a first, a second and a third distance or movement sensor, the housing having an outer surface and being configured to be positioned in the cavity, the distance or movement sensors being configured to sense a distance or velocity from, respectively, a first, a second, and a third positions, and in, respectively, a first, a second and a third directions and output a corresponding first, second and third output signals, respectively, The system further comprises a controller configured to determine, from the first, second and third output signals, information relating to distances and/or relative movements between the sensor and the cavity along the first, second and third directions, respectively. It is further described the associated method.

## Description

The present invention relates to the determination of a parameter related to the shape or blood perfusion of a body cavity such as an ear canal.

In a first aspect, the invention relates to a system for deriving information relating to a cavity in blood perfused tissue comprising a cavity having an inner surface, the system comprising:
- a sensor comprising a housing and, in or at the housing, a first, a second and a third distance or movement sensor, the housing having an outer surface and being configured to be positioned in the cavity,
- the first distance or movement sensor being configured to sense a first distance or velocity from a first position and in a first direction and output a corresponding first output signal,
- the second distance or movement sensor being configured to sense a first distance or velocity from a second position and in a second direction and output a corresponding second output signal,
- the third distance or movement being configured to sense sensing a first distance or velocity from a third position and in a third direction and output a corresponding third output signal, where, when projected on to a predetermined plane:
   ∘ a first tangent exists to the outer surface and intersecting the first radiation,
   ∘ a second tangent exists to the outer surface and intersecting the second radiation,
   ∘ a third tangent exists to the outer surface and intersecting the third radiation,
   ∘ the first, second and third tangents define a closed figure inside which at least 50% of the housing exists,
- a controller configured to determine, from the first, second and third output signals, information relating to distances and/or relative movements between the sensor and the cavity along the first, second and third directions, respectively.

In the present context, the system comprises the sensor with the at least three distance or movement sensors and the controller. Naturally, the system may comprise additional elements. In one situation, the system would be for use as a hearable, hearing aid, ear bud or the like. Then, all other typical elements, such as filters, amplifiers, microphones, controllers, signal receivers and the like, usual for hearables may be provided if desired.

The controller may be fully or partly provided in the housing or may be adjacent thereto, such as in a system housing in which the sensor housing is also positioned. Alternatively, the controller may be remote to the sensor, where the sensor and controller may exchange data via a remote connection, wirelessly and/or wired. The same controller may be used for multiple sensors or systems if desired.

The controller is configured to receive the output signals of the distance or movement sensors and determine the information. The controller may be used for controlling also other portions of the system, such as other elements in the housing if desire and if present.

The cavity may be any cavity inside a human or animal, such as an ear canal, oral cavity or the like.

A blood perfused part, such as of the ear canal, may be any part of the ear canal, as all parts thereof are perfused by blood so as to be supplied by blood. Usually, blood perfusion is a transport of the blood in blood vessels of tissue, such as arteries, veins, alveoli or the like. Due to the perfusion, the blood is moving in the tissue of the part. Often, the portion of the blood perfused part receiving and emitting radiation is so small, that the blood may be said to have a single direction of movement. However, for this measurement, the blood may flow in multiple directions.

Usually, the blood perfused part is close to a surface part of the ear canal. This may be in the outer portions of the ear canal or in the bony portion thereof.

The inner surface would normally be at an interface between the tissue and air, such as where skin is normally provided.

The housing is configured to be provided in the cavity. In one embodiment, the housing may be completely provided inside an ear canal. A housing may be custom made to fit a particular cavity so as to contact the inner surface over extensive portions of the housing, such as over positions at least 90% of a circumference of the housing around a longitudinal axis of the cavity. In some situations, it is desired that the housing contacts the cavity, such as at positions where the directions cross the inner surface. In other situations, the housing may be provided in the cavity in a position where no portion of the housing touches the cavity, at least at some points in time. Portions of the cavity may deform when the person is chewing, running, or the like and during such situations, the housing may touch the cavity.

A first, a second and a third distance or movement sensors are provided. Naturally, additional distance or movement sensors may be provided. The distance or movement sensors of the system may be of the same type or of different types. Different types are described further below.

Each distance or movement sensor is configured to sense a distance or velocity from a position and in a direction and output a corresponding output signal. A sensor may be a contact sensor configured to contact the housing and the inner surface to determine the distance and/or movement. alternatively, the sensor may be a contact-less sensor such as an optical sensor or a sensor operating by the use of airborne signals, such as sound. When the cavity is an ear canal, it may be desired that the sensor is optical or uses non-audible sound.

The first, second and third distance or movement sensors have first, second and third directions, which are positioned at positions of the housing and thus a position of the outer surface of the housing exists from which the direction extends. The distance or movement sensors may be positioned at these positions if desired.

At the position from which a direction extends, a tangent may be defined, when the housing surface, the position and the direction are projected on to a desired plane, such as a plane perpendicular to a longitudinal direction of the sensor, so that:
∘ a first tangent exists to the outer surface and intersecting the first radiation,
∘ a second tangent exists to the outer surface and intersecting the second radiation,
∘ a third tangent exists to the outer surface and intersecting the third radiation,
∘ the first, second and third tangents define a closed figure inside which at least 50% of the housing exists.

In the present context, the at least 50% of the housing would be at least 50% of a cross-sectional area of the housing in the plane or an outline of the housing when projected on to the plane. Thus, when at least 50% of the housing, such as at least 75% of the housing, such as at least 95% of the housing, in the cross section in the plane, the positions and the directions are sufficiently independent for a good determination of the relative movement

The controller is configured to determine, from the first, second and third output signals, information relating to relative movements between the sensor and the cavity along the first, second and third directions, respectively. This determination can be based directly on the output from the distance or movement sensors.

It may be desired that the controller is instead or additionally capable of deriving, from the distances and/or relative movements, information relating to a shape of the cavity, a variation of the shape of the cavity, and/or a relative movement or distance between the sensor and the cavity. Clearly, from the distances and relative movements, a number of parameters may be determined. The sensor may be stationary within the cavity which deforms, whereby the deformation may be determined from the relative movement. If the cavity is not moving, its shape may be determined from the distances. If relative movement is determined, the sensor may move relative to the cavity and/or the cavity may change shape.

A second aspect of the invention relates to a system for deriving a shape or a variation of the shape of a cavity in blood perfused tissue comprising a cavity having an inner surface, the system comprising:
- a sensor comprising a housing and, in or at the housing, a first, a second and a third distance or movement sensor, the housing having an outer surface and being configured to be positioned in the cavity,
- the first distance or movement sensor being configured to sense a first distance or velocity from a first position and in a first direction and output a corresponding first output signal,
- the second distance or movement sensor being configured to sense a first distance or velocity from a second position and in a second direction and output a corresponding second output signal,
- the third distance or movement being configured to sense sensing a first distance or velocity from a third position and in a third direction and output a corresponding third output signal,
   o where, when projected on to a predetermined plane, no angle being 180° or more exists between adjacent directions,
- a controller configured to determine, from the first, second and third output signals, information relating to relative movements between the sensor and the cavity along the first, second and third directions, respectively.

In this context, the system, sensors and the like may be as described above. Also, the plane and directions may be as described.

According to this aspect, however, the directions are determined so that, when projected on to a predetermined plane, no angle being 180° or more exists between adjacent directions,

Again, the controller is configured to determine, from the first, second and third output signals, information relating to distances and/or relative movements between the sensor and the cavity, such as along the first, second and third directions, respectively.

In this context, in the plane, the directions are projected on to the plane. An angle between two directions is seen by extending the directions until they intersect. The angle is determined between adjacent directions. No angle between adjacent directions is 180° or more, such as 160° or more, such as 140° or more.

All directions may be extended so as to each intersect at least one other of the directions. From this, the angles between adjacent directions may be identified.

Alternatively, the directions may, in the plane, be translated to all intersect in a single point, so that adjacent directions are easily determined.

The above considerations may be made, for selection of the directions, for any plane, such as a plane perpendicular to the above plane and/or a plane comprising or being parallel to the longitudinal axis of the housing or the cavity. In an especially interesting embodiment, this plane may be vertical or intended to be vertical. In some situations, the housing is not rotationally symmetric so that one direction thereof is intended to be vertical, when the user has a particular position, such as when a person is standing up and looking straight forward with a level head. In some situations, the housing is connected to a cable for transporting power, signals or the like to and from another element, such as a so-called BTE, which is an element designed to be positioned behind the ear of a person and which, by the cable, is connected to the housing then configured to be positioned in an ear canal of that ear. In that situation, the cable and the connection thereto, and optionally also the other element, will define a rotation of the housing inside the ear canal, and thus which direction is intended to be vertical.

All embodiments, situations, and considerations of one aspect of the invention may be equally relevant for other aspects of the invention.

In one embodiment, the controller is configured to, in the deriving step, determine that:
- a first distance from the housing to the inner surface along the first direction,
- a second distance from the housing to the inner surface along the second direction, and
- a third distance from the housing to the inner surface along the third direction,
all have varied within the predetermined period of time.

In this manner, a variation in the shape of the cavity may be varying and/or a relative position of the housing in the cavity and/or a distance between the housing and the cavity may be varying. This may be for a number of reasons, also depending on the period of time.

A pulse will make blood perfused tissue swell and contract, which may alter the shape of the cavity. The period of time and the frequency of this pulse will be well-known. An activity of the person or animal may make the sensor position shift in the cavity. The time period and the frequency hereof is also well-known. Yet another situation would be the long term effect of calcification or aging of blood vessels. With age, blood vessels become more stiff, whereby the pulse and blood pressure caused by the action of the heart will over time make the tissue swell less. Thus, over time, calcification or aging of the blood vessels may be visible from the deformation of the cavity caused by the pulse. The time period of this effect is clearly much longer than those of the other examples.

In one situation, the controller is configured to, in a deriving step, derive information relating to a variation, within a predetermined period of time and in the plane, of an area encircled by the inner surface of the cavity. This may be a variation for all distances which is a reduction or an increase. Thus, velocity sensors or position sensors could all have an in-phase component indicating common increase or decrease of the distances.

In that or another situation, the controller is configured to, in a deriving step, derive information relating to a deformation, in the plane, of the inner surface of the cavity. In this situation, distances along one or more directions may decrease and those along other direction may increase. In this situation, position or velocity sensors may have out-of-phase components indicating such increases and decreases of distances.

In one situation, the controller is configured to, in a deriving step, determine a relative movement, in the plane, of the housing in relation to the inner surface of the cavity. This may be seen during e.g. an activity of the person or animal such as running, chewing, talking, dancing and the like. Naturally, this relative movement may be compared to a rest position, such as a position determined from a weighted sum of determined positions.

In one situation, the controller is configured to, in a deriving step, determine a period of the relative movements. A period or periodicity is seen in repeated movements, such as caused by a pulse, walking, chewing, swallowing, running or the like.

It may in addition or alternatively be desired to determine an amplitude of the relative movements.

In one embodiment, the system further comprises a resilient element and wherein at least one of the first, second and third distance or movement sensor is configured to output an output signal relating to a distance, along the pertaining direction, between the pertaining sensor and the resilient element.

The resilient element may be configured to fix the system or sensor in relation to the cavity. In hearables and hearing aids, a suitable resilient element may be a so-called dome, which is attached to the sensor or housing and extends therefrom. Domes are made of materials which are compliable and which are suitable for contact with the tissue of the cavity.

Preferably the resilient element is configured to engage the inner surface of the cavity. A dome is configured to engage the ear canal to fix itself in relation to the ear canal.

When the at least one of the distance or movement sensors is configured to output the output signal relating to the distance to the resilient element, this distance need not be to the inner surface. This is especially interesting when the distance or movement sensor is a contact sensor or an optical sensor or a sound based sensor, as the contact, radiation or sound may then be launched toward the resilient element and not the tissue of the cavity.

In addition, the resilient element may be adapted to the sensor type. If the sensor type is a contact type, the resilient element may be provided sufficiently rugged to not be damaged by the contact with the sensor. If the sensor is an optical sensor, the reflection, reflectivity, scattering, absorption and the like of the material or the surface of the resilient element may be adapted to the optical sensor. In addition, launching an optical beam on to the resilient element and not the tissue of the cavity makes it possible to use more wavelengths or a higher power which would otherwise damage the tissue.

In one embodiment, at least one of the first, a second and a third distance or movement sensors comprises Doppler-based sensor. A Doppler-based sensor is a sensor outputting a wave, such as sound or radiation, which wave is reflected by the surface or tissue (or resilient element) and reverted to the sensor. The received wave will have experienced a frequency (wavelength) shift if the distance between the sensor and the portion of the tissue (resilient element) changes. Thus, a relative velocity may be determined.

In one situation, the Doppler-based sensor comprises a VCSEL with integrated Photodetector configured to launch radiation from the pertaining position and in the pertaining direction, receive radiation from the pertaining direction and output a pertaining output signal.

In the present context, a VCSEL is a Vertical-cavity surface-emitting laser and a VCSEL with integrated photodetector is a very compact element well suited for use in space critical situations. A Vertical-cavity surface-emitting laser, or VCSEL, is a laser having an optical cavity in which the radiation is created and from which the radiation is emitted. The built-in photodetector, which could be of any type, such as a photodiode, photo resistor, CCD, phototransistor or the like, is positioned so as to detect radiation, such as generated radiation, in the laser/optical cavity. The active region of the laser is provided in the optical cavity and may constitute the optical cavity. Two reflecting elements, often Bragg reflectors, are provided on opposite sides of the optical cavity or active region. Other optical components, such as lenses, slits, reflectors or the like, may be provided outside of the VCSEL. Clearly, the VCSEL with integrated Photodetector may itself be exposed to the surroundings of the system, such as if provided on the housing or in an opening or cavity thereof, or it may be provided in the housing and emitting/receiving the radiation through a window. If additional components are provided they may, in addition to the real purpose thereof, also protect the laser. In one situation, a light guide, such as a flexible light guide, may be provided between the laser, provided inside the system housing, and a surface thereof.

The vertical-cavity surface-emitting laser (VCSEL) usually is a type of semiconductor laser diode with laser beam emission perpendicular from the top surface, contrary to conventional edge-emitting semiconductor lasers (also in-plane lasers) which emit from surfaces formed by cleaving the individual chip out of a wafer.

Self-mixing interferometry (SMI) is a measurement technique, in which a laser beam is reflected from an object and fed back into the laser. The reflected light interferes with the light generated inside the laser, and this causes changes in the amplitude and frequency of the output of the laser. Information about the target object, like position and velocity can be obtained by analysing these changing properties, which are picked up by or represented in the radiation reaching the photodetector.

In that or another embodiment, the at least one of the first, a second and a third distance or movement sensors further comprises a direction sensor configured to sense a direction of a relative movement between the inner surface and the pertaining sensor along the pertaining direction. A direction sensor could be combined with a Doppler-based sensor, as Doppler-based sensors may be able to quantify a relative velocity but not the direction of the velocity. A direction sensor could complement a Doppler-based sensor so that knowledge would be created to both the velocity and the direction thereof.

A direction sensor could e.g. be a capacitive sensor or an optical sensor. A simple optical sensor would be a photodetector sensing a change in an intensity of radiation reflected from the tissue. If the intensity drops, the distance increases and vice versa.

In one situation, at least one of the first, a second and a third distance or movement sensors comprises time-of-flight-based sensor. Sensors of this type may be distance sensors. A signal is launched toward the target (tissue/surface) where it is reflected and received by the sensor. The distance is correlated to the time of flight of the signal. Sensors of this type may be based waves such as sound or radiation. A sequence of such distance measurements may form a determination of a velocity, acceleration or the like.

A suitable time-of-flight based sensor may comprise a VCSEL with integrated Photodetector and a drive current supply configured to supply the VCSEL with integrated Photodetector with a varying drive current, the VCSEL with integrated Photodetector configured to launch radiation from the pertaining position and in the pertaining direction, receive radiation from the pertaining direction and output a pertaining output signal. This is described further below.

A third aspect of the invention relates to a method of deriving information relating to a cavity in blood perfused tissue comprising a cavity having an inner surface, the method comprising:
- providing a sensor in the cavity, the sensor comprising a housing and, in or at the housing, a first, a second and a third distance or movement sensor, the housing having an outer surface,
- the first distance or movement sensor sensing a first distance or velocity from a first position and in a first direction and outputting a corresponding first output signal,
- the second distance or movement sensor sensing a first distance or velocity from a second position and in a second direction and outputting a corresponding second output signal,
- the third distance or movement sensor sensing a first distance or velocity from a third position and in a third direction and outputting a corresponding third output signal, where, when projected on to a predetermined plane:
   ∘ a first tangent exists to the outer surface and intersecting the first radiation,
   ∘ a second tangent exists to the outer surface and intersecting the second radiation,
   ∘ a third tangent exists to the outer surface and intersecting the third radiation,
   ∘ the first, second and third tangents define a closed figure inside which at least 50% of the housing exists, and
- determining, from the first, second and third output signals, information relating to distances and/or relative movements between the sensor and the cavity along the first, second and third directions, respectively.

As mentioned above, all aspects, embodiments, situations and considerations mentioned in relation to one aspect are equally relevant to the other aspects of the invention.

The sensors, system, controller, directions, tangents and the like are as described above.

A fourth aspect of the invention relates to a method of deriving information relating to a shape or a variation in the shape of a cavity in blood perfused tissue comprising a cavity having an inner surface, the method comprising:
- providing a sensor in the cavity, the sensor comprising a housing and, in or at the housing, a first, a second and a third distance or movement sensor, the housing having an outer surface,
- the first distance or movement sensor sensing a first distance or velocity from a first position and in a first direction and outputting a corresponding first output signal,
- the second distance or movement sensor sensing a first distance or velocity from a second position and in a second direction and outputting a corresponding second output signal,
- the third distance or movement sensor sensing a first distance or velocity from a third position and in a third direction and outputting a corresponding third output signal,
   o where, when projected on to a predetermined plane, no angle being 180° or more exists between adjacent directions,
- determining, from the first, second and third output signals, information relating to relative movements between the sensor and the cavity along the first, second and third directions, respectively, and
- deriving, from the relative movements, the information relating to the shape or the variation in shape of the cavity.

The system, sensors, directions, angles and the like may be as described above.

In one embodiment, the method further comprises the step of deriving, from the distances and/or relative movements, information relating to a shape of the cavity, a variation of the shape of the cavity and/or a relative movement or position between the sensor and the cavity.

In one embodiment, a deriving step comprises determining that:
- a first distance from the housing to the inner surface along the first direction,
- a second distance from the housing to the inner surface along the second direction, and
- a third distance from the housing to the inner surface along the third direction,
all have varied within the predetermined period of time.

In one embodiment, a deriving step comprises deriving information relating to a variation, within a predetermined period of time and in the plane, of an area encircled by the inner surface of the cavity.

In one embodiment, a deriving step comprises deriving information relating to a deformation, in the plane, of the inner surface of the cavity.

In one embodiment, a deriving step comprises deriving a relative movement, in the plane, of the housing in relation to the inner surface of the cavity.

It may be desired that a deriving step comprises determining a period and/or an amplitude of the relative movements.

It one embodiment, the sensor comprises a resilient element provided between the cavity and at least one of the first, second and third distance or movement sensor, wherein the pertaining output signal relates to a distance, along the pertaining direction, between the resilient element and the pertaining distance or movement sensor.

Then, the resilient element may engage the inner surface of the cavity.

In one embodiment, at least one of the first, a second and a third distance or movement sensors comprises Doppler-based sensor.

Then, the Doppler-based sensor could comprise a VCSEL with integrated Photodetector launching radiation from the pertaining position and in the pertaining direction, receiving radiation from the pertaining direction and outputting a pertaining output signal.

It may be desired that the at least one of the first, a second and a third distance or movement sensors further comprises a direction sensor sensing a direction of a relative movement between the inner surface and the pertaining sensor along the pertaining direction.

In one embodiment, at least one of the first, a second and a third distance or movement sensors comprises time-of-flight-based sensor.

Then, the time-of-flight based sensor comprises a VCSEL with integrated Photodetector supplied by a varying drive current, the VCSEL with integrated Photodetector launching radiation from the pertaining position and in the pertaining direction, receiving radiation from the pertaining direction and outputting a pertaining output signal.

In the following, preferred embodiments will be described with reference to the drawing, in which:
- Figure 1 illustrates a first embodiment of a system according to the invention inside a cavity,
- Figure 2 illustrates primary elements of a VCSEL with integrated photodetector,
- Figure 3 illustrates the use of a time-of-flight based or Doppler-based sensor for determining a parameter,
- Figure 4 illustrates relative movement between the sensor and the cavity,
- Figure 5 illustrates swelling and contraction of the cavity,
- Figures 6 and 7 illustrate different types of deformation of the inner surface of the cavity,
- Figure 8 illustrates determination of swelling of the tissue,
- Figure 9 illustrates determination of relative movement between the sensor and the cavity,
- Figure 10 illustrates determination of the deformation of figure 6,
- Figure 11 illustrates determination of the deformation of figure 7,
- Figure 12 illustrates tilting of the sensor relative to the cavity,
- Figure 13 illustrates the use of a resilient element,
- Figure 14 illustrates the plane with the tangents and
- Figure 15 illustrates angles between the directions.

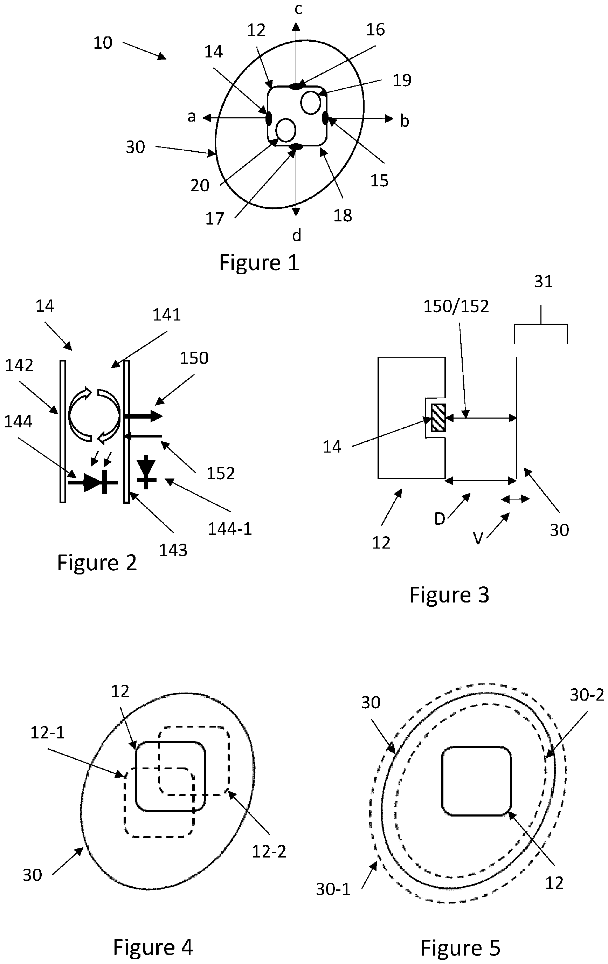

In figure 1, a system 10 is illustrated provided inside a cavity comprising blood perfused tissue 31 (see figure 3) and having an inner surface 30. Figure 1 is seen in a cross section though the cavity and the sensor 12, where 4 distance or movement sensors 14, 15, 16, and 17, are seen, each sensing a distance and/or velocity/movement along one of the directions a, b, c and d, respectively and outputting a corresponding signal. The sensor 12 has a housing 18 and the individual distance or movement sensors may be provided on or in the housing 18. The distance or movement sensors are configured to determine a distance to or movement in relation to the cavity along the pertaining direction

Further, a processor 19 is provided, often in the housing 18, for receiving output signals from the distance or movement sensors and for outputting a parameter or information from which the shape or shape variation of the cavity or more precisely its inner surface 30, or a position of the sensor in the cavity, or a distance between the sensor and the cavity or relative movement between the sensor and cavity may be determined or estimated. The processor 19 may be configured to communicate with other electronic components via a connector, communication port, wirelessly or via or cable (not illustrated), which may also carry power and/or other signals for the sensor 12, a microphone (not illustrated) or a sound generator 20, if present, or the like. Alternatively, a battery may be provided in the housing for powering the individual elements.

Naturally, the sensor 12 may be provided with a sound generator 20 so as to function as a hearable, hearing aid or the like. Alternatively, the sensor 12 may comprise a sound input for receiving sound from an external sound generator and for transporting that sound to a sound output of the housing 18.

One suitable type of distance or movement sensor is, cf. figure 2, a VCSEL with Integrated Photodetector 14 which may be a Vertical Cavity Surface Emitting Laser with Integrated Photodetector 144. A vertical-cavity surface-emitting laser (VCSEL) is a type of semiconductor laser diode with laser beam emission perpendicular from the top surface, contrary to conventional edge-emitting semiconductor lasers (also in-plane lasers) which emit from surfaces formed by cleaving the individual chip out of a wafer. We note that the term VCSEL includes Vertical external cavity surface emitting lasers (VECSEL).

The VCSEL with integrated Photodetector is positioned to output the radiation and receive radiation from the same direction - the direction of the distance or movement sensor.

In figure 2, a VCSEL with integrated photodetector 14 is seen having an active region or optical cavity 141 provided between two reflecting portions 142 and 143, often formed by Bragg reflectors. Radiation 150 is emitted from the optical cavity 141 through the reflecting portion 143, which is not completely reflecting. Radiation 152 received into the cavity 141 will interfere with the operation of the VCSEL in a manner which will be detectable by the detector 144, cf below.

Clearly, the sensor or detector 144 could be positioned suitably in relation to the VCSEL, but when it is monolithically integrated with the VCSEL, a very compact solution is obtained which may be particularly relevant when space is an issue, such as when the hearable is for use in an ear canal of a person.

A VCSEL with integrated Photodetector may be employed in a number of manners as will become clear below. A VCSEL with integrated Photodetector has a number of advantages, such as small size, rugged, small power consumption, highly directive sensing, longer range sensing is possible and the like.

A manner of determining a parameter relating to a distance, D, to, or a variation in the distance to (relative movement, V, between the sensor and the surface) the surface 30 or tissue 31, is seen in figure 3.

Two overall types of sensors 14 may be used: movement sensors such as velocity sensors or acceleration sensors and distance sensors.

A movement sensor may be a velocity or speed sensor or an acceleration sensor. A movement sensor may comprise a distance sensor, as described further below, and perform a sequence of distance measurements. The variation in the distance over time may be converted into a velocity/speed and/or acceleration.

Other types of velocity/speed/acceleration sensors are based on the Doppler effect. In such sensors, a signal, such as a predetermined frequency, is output from the sensor to the tissue/surface which reflects or scatters the signal back to the sensor. If a relative movement takes place between the sensor and the tissue/surface along the direction in question, the frequency of the scattered/reflected signal will be shifted. This shift is determined by the relative velocity between the sensor and the tissue/surface.

Doppler based sensors may operate on any type of radiation or sound.

A suitable Doppler based sensor is the above-mentioned VCSEL with integrated Photodetector. The operation of a VCSEL with integrated Photodetector for determination of blood flow parameters may be seen in Applicant's co-pending application filed on even date and with the title: "A system and a method of determining a parameter related to blood flow in a blood perfused part". In this application, the radiation 150 is launched on to the surface 30 of a tissue portion 31, such as an ear canal, of the person. Inside the tissue 31, such as the ear canal, blood is flowing. In blood, different constituents exist, such as red blood cells. The flood flows in arteries, veins, capillaries in the tissue. Thus, a Doppler based measurement may be used for determining a blood flow parameter.

A VCSEL with integrated Photodetector may be used for other purposes, for example as a movement sensor.

In general, a VCSEL with integrated Photodetector launches the radiation 150 output toward an area or volume of the tissue and surface from which part of the radiation is reflected or scattered causing a wavelength shift of the radiation 152 received back into the cavity 141 and the photodetector 144 will output a signal from which the relative velocity may be determined.

It is noted that the frequency shift of the laser may be both upwards and downwards in freqeuency, and it may not be possible to discern those two situations from each other in the signal from the photodetector 144. Thus, it may be desired to use the VCSEL with integrated Photodetector, or other Doppler based sensors, together with a sensor configured to determine a direction of the relative movement. A sensor of that type may be a position sensor or other type of sensor. In one example, another photodiode 144-1, not integrated in the VCSEL, may be provided for determining an intensity of the radiation 152. This intensity will depend on the distance to the tissue/surface, so that if the intensity increases, the distance reduces, and vice versa.

A simpler movement sensor may simply launch radiation on to the surface or tissue and determine an intensity of reflected radiation. This intensity may correlate to the distance, so that a change in intensity will indicate a speed or velocity of a relative movement between the sensor and the irradiated tissue/surface.

Another type of distance sensor is based on a VCSEL with integrated Photodiode which is operated in a particular manner so that the wavelength output thereby varies over time. Then, the wavelength of the radiation 150 will differ from that of radiation 152 due to the variation in wavelength. The wavelength variation may be caused by varying the drive current to the VCSEL. This causes the radiation reflected from the cavity/surface to differ from that presently output by the VCSEL. This difference will depend on (twice) the distance and the degree of variation of the wavelength and thus the drive current. The wavelength variation, and thus the distance, may be determined from the resulting output of the photodetector, as the self-mixing in the cavity will be affected, so that the photodetector signal will have a frequency offset (which is proportional to the time-of-flight). For example, in a situation with no relative movement between the sensor and the cavity/surface, the output frequency will be non-zero. With increasing or decreasing distance, the output frequency will increase or decrease, depending whether the laser wavelength is increasing or decreasing over time.

Naturally, a relation between the drive current and the wavelength would be useful in the above determination.

Clearly, multiple determinations may be made over time, so that a velocity and the like may be determined. It may be desired to synchronize signal acquisition with the variation in the drive current of laser.

A distance sensor may be a time-of-flight sensor or a radiation sensor determining incoming radiation from the tissue 31 and/or surface 30 and determining or estimating the distance based on an intensity of the received radiation. A distance sensor may thus comprise a radiation source configured to direct radiation toward the tissue or surface, and the radiation sensor may be configured to receive radiation from the surface/tissue along the direction of the sensor. A particular example is given above by the VCSEL with integrated Photodetector operated to vary its output wavelength.

A time-of-flight based sensor may output a signal, such as radiation or sound, which is reflected or scattered by the surface or tissue and reverted to the sensor. The distance to the tissue/surface will then be determinable from the time of flight and the velocity of the signal.

For a number of reasons, which will be described further below, the surface 30 may move toward and away from the sensor 12, such as at an average velocity V, which may vary over time in amplitude and direction.

Clearly, the volume of the tissue 31 and thus the position of the surface 30 will depend on the heartbeat of the person. Also, the stiffness of the blood vessels will affect the velocity of the surface 30, as the velocity will be higher in stiffer blood vessels not able to expand to the same degree to compensate for an increasing blood pressure due to the heart pumping.

When the distance and/or movement sensors are sufficiently many and are directed in sufficiently different directions, the distance/movement measures thereof may be used for determining certain parameters of the cavity, the shape of the cavity, variation in the shape of the cavity and/or the position or a variation thereof of the sensor housing in the cavity.

In figure 4, different positions, 12, 12-1 and 12-2, of the sensor 12 are illustrated within the surface 30. Movement of the housing inside the cavity may be caused by a wearer moving, such as running.

In figure 5, a contraction and widening of the cavity is seen with different surface positions 30-1 and 30-2, where the surface 30 could be a rest position or a mean position. The widening and contraction of the cavity may be caused by the action of the pulse, so that the swelling and contraction of the tissue is caused by the blood pressure variations caused by the heartbeat.

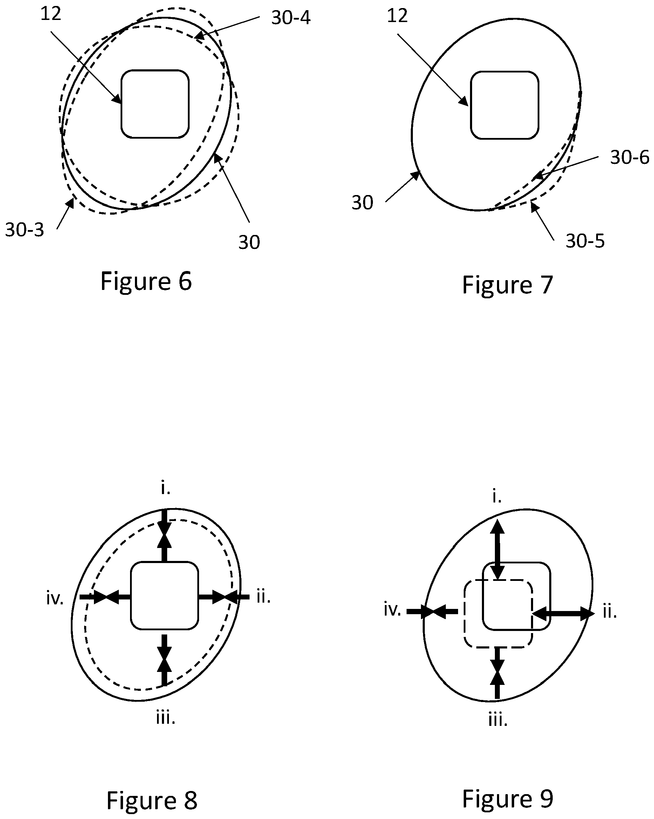

Figure 6 illustrates deformation of the surface 30 between a rounder shape 30-4 and a more elliptical shape 30-3. Deformation may be seen in a number of situations, such as when the person is chewing or even moving his/her eye.

Figure 7 illustrates another more localized deformation where the surface has a portion altering between the shapes 30-5 and 30-6.

The determination of the deformations or movements may be obtained in different manners.

In figure 8, determination is illustrated of swelling of the tissue, as seen in figure 5, and/or the reduction of the area of the cavity in a plane perpendicular to an axis of the sensor and/or of the cavity. When all 4 distances along the directions i, ii, iii, iv have the same phase (all decreasing), the individual distances may be used for determining the swelling of the tissue. A period of the phase is indicative of the pulse rate. Naturally, displacements and other error sources may be addressed by determining average distances and the like.

In figure 9, the situation is illustrated where the sensor 12 moves relatively to the cavity surface 30. In this situation, directions ii. and iv. will have opposite phases and the same will be the situation for directions i. and ii.

In the drawings, arrows directed toward each other indicate distance reduction and arrows directed away from each other indicate distance increase.

Clearly, combinations of the movements may be seen. If directions ii. and iv. have opposite phases but not the same sizes, the sensor 12 is moving in the cavity but the shape of the cavity also changes.

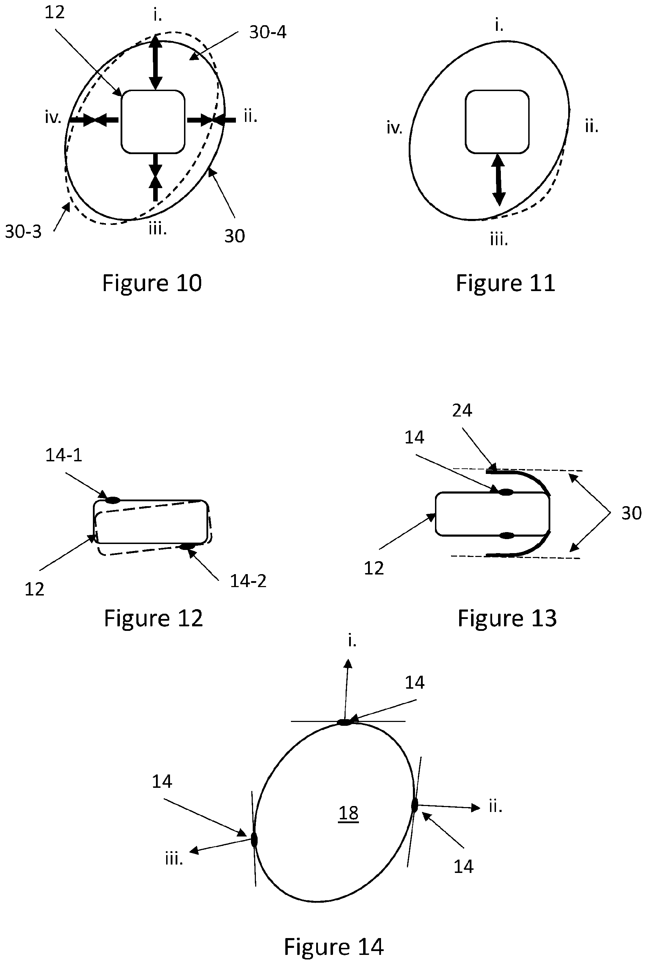

In figure 10, the situation of figure 6 is seen where the cavity is deformed from a rounder shape 30 to a more elliptical shape 30-3. Here, it is seen that the direction i. increases but the directions ii., iii. and iv. decrease.

In figure 11, the situation of figure 7 is seen where a local deformation is seen increasing the distance in direction iii. but leaving the other distances unaltered.

Naturally, not only can the cavity change shape in many different ways, the sensor may also displace within the cavity in many ways depending on how the sensor is provided in the cavity, such as if the sensor is fastened or fixed inside the cavity. In one situation, the cavity may be an oblong cavity, such as an ear canal, where the sensor is fixed inside the cavity at one end, such as by using a soft dome attached to the sensor housing and configured to engage or touch the inner surface. Often, the dome will be attached at one end of the sensor fixing the position of that end better than the opposite end in relation to the cavity.

The periodicity of the determined parameters for swelling, movement and deformation can have a specific time dependent pattern related to the wearers activity or to a biometric parameter. Knowledge of these patterns may contribute to distinguishing of the parameters for swelling, movement and deformation. For example, a heart beat will cause a the tissue to first swell and then contract. This movement will follow a particular pattern which may be recognized. Also, the relative movement of the sensor in the cavity when the user runs will be determinable.

Actually, from a number of these periodic or substantially periodic situations, the direction of the relative movement may be known in advance so that the direction of the movement need not be determined separately, such as when using a Doppler based sensor. When the user runs, the downward movement will be more forceful and thus swifter than the upward movement. The swelling caused by a heart beat will be swifter than the following contraction of the tissue. Then, from such information, the direction of the movement may be determined without having to be determined directly.

In figure 12, the sensor 12 is seen rotating around an axis provided around the right end thereof, so that the largest displacement is at the left end.

Thus, as the largest displacement is expected at the right end, the sensors 14-1 may be desired provided at the left end in order to be able to determine the distance or relative movement, vis-à-vis the cavity, with a higher precision. Naturally, sensors 14-2 may be provided anywhere and also at the right end.

Even though the sensors and directions, distances and the like above have been described above as projected on a plane, such as a plane perpendicular to an axis, such as a longitudinal axis, of the sensor 12 and/or the cavity, the positions of the individual sensors 14 in relation to the housing 18, in any desired manner. It may be preferred to provide all sensors in the same plane perpendicular to the longitudinal axis, but this may not be possible or practical due to the positions and space requirements of other elements of the sensor 12, such as a sound generator.

It may be preferred to provide sets of sensors 14, directed as described above and below, in different portions of the sensor 12 so as to be able to determine the shape or shape variation of the cavity at different positions, such as along a longitudinal direction of the cavity, or to determine different distances or relative movements of the different portions of the sensor vis-à-vis the cavity.

In order for the sensors 14 to output signals from which a suitable shape, variation or the like, the positions thereof should be selected with some thought.

Figure 14 illustrates a more irregularly shaped housing 18 with three sensors 14 with the directions i., ii. and iii.

Tangents are illustrated to the housing surfaces at the positions of the sensors 14.

When the tangents define a closed figure (a triangle in this situation), at least 50% of the cross sectional area of the housing 18 should be inside this figure. In this manner, the positions of the sensors 14 is suitable.

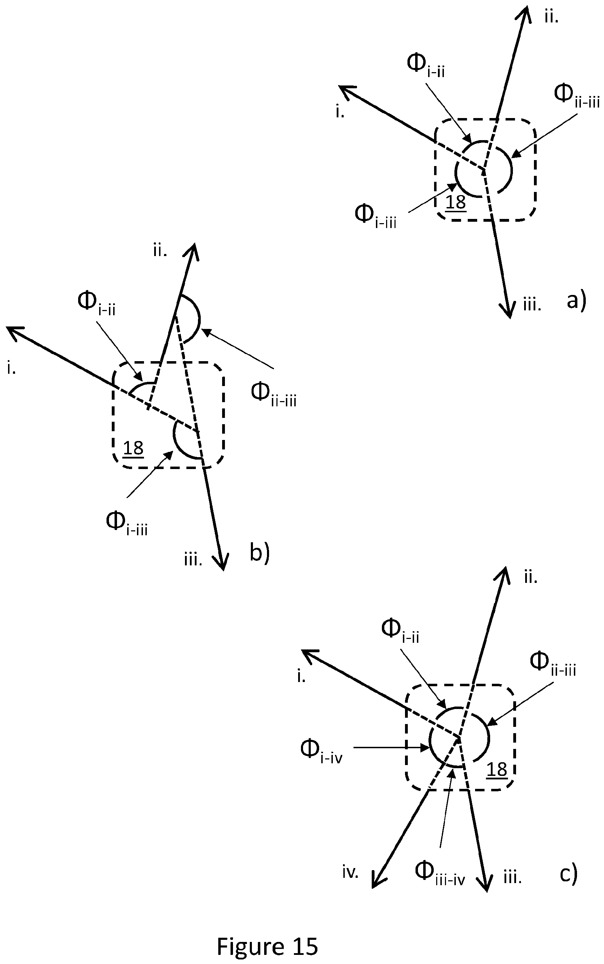

In another situation, seen in figure 15, suitable positions may be determined instead by the directions of the sensors 14. In figure 15, the directions are represented by arrows pointing from the surface of the housing towards the cavity surface. The arrows are extended with dashed lines until intersect one another. These directions are then illustrated in a plane, such as a plane perpendicular to a longitudinal axis of the housing or the cavity.

In illustration a) 3 directions, i., ii. and iii. are illustrated. The corresponding sensors may be positioned in the housing 18 or on or at the surface thereof. The sensors may be of any of the above-mentioned types. Clearly, the sensors may be of different types. The direction of each sensor is determined by the sensor, such as a direction thereof or one or more elements, such as a laser, lens, antenna, sound generator or the like.

Angles between the directions are indicated by means of circle segments. The angles between adjacent directions are indicated by a letter Greek capital phi. The angles are illustrated between adjacent directions. In this context, the angle is the smallest angle between the two directions.

According to one aspect of the invention, the largest of the angles is smaller than 180°. In this manner, the directions are sufficiently diverse to allow a suitable determination of the parameters sought.

In illustration b), the positions of some of the sensors are altered, maintaining the same directions, so that the lines do not intersect in a single point. In this situation, the angles will be the same. The angles may be determined at the intersections between the directions, or the directions may be translated to intersect at a particular position such as at a central position of the cross section of the housing.

In illustration c) four directions i., ii., iii. and iv. are seen. The angles are also illustrated to display that the angles may be determined for any number of directions exceeding 2.

Figure 13 illustrates the use of a resilient element, such as a dome 24 attached to an end of the sensor 12. In this embodiment, the sensors 14, or some of the sensors 14, are positioned so as to determine the distance/velocity/movement in a direction toward an inner surface of the dome.

When the dome contacts the inner surface 30, the distance from the sensor 14 to the surface 30 may be estimated, and any relative movement between the surface 30 and the inner surface of the dome may be assumed to be the same.

The use of a resilient element may bring about a number of advantages. The properties of the resilient material may be adapted to the sensor type. The reflecting or scattering properties may be adapted to an optical sensor, for example. Also, for optical sensors, the radiation wavelength and intensity may be selected more freely when the radiation is not launched on to the tissue of the cavity.

## Claims

1. A system for deriving a shape or a variation of the shape of a cavity in blood perfused tissue comprising a cavity having an inner surface, the system comprising:
- a sensor comprising a housing and, in or at the housing, a first, a second and a third distance or movement sensor, the housing having an outer surface and being configured to be positioned in the cavity,
- the first distance or movement sensor being configured to sense a first distance or velocity from a first position and in a first direction and output a corresponding first output signal,
- the second distance or movement sensor being configured to sense a first distance or velocity from a second position and in a second direction and output a corresponding second output signal,
- the third distance or movement being configured to sense sensing a first distance or velocity from a third position and in a third direction and output a corresponding third output signal, where, when projected on to a predetermined plane:
∘ a first tangent exists to the outer surface and intersecting the first direction,
∘ a second tangent exists to the outer surface and intersecting the second direction,
∘ a third tangent exists to the outer surface and intersecting the third direction,
∘ the first, second and third tangents define a closed figure inside which at least 50% of the housing exists,
- a controller configured to determine, from the first, second and third output signals, information relating to distances and/or relative movements between the sensor and the cavity along the first, second and third directions, respectively.

2. A system for deriving a shape or a variation of the shape of a cavity in blood perfused tissue comprising a cavity having an inner surface, the system comprising:
- a sensor comprising a housing and, in or at the housing, a first, a second and a third distance or movement sensor, the housing having an outer surface and being configured to be positioned in the cavity,
- the first distance or movement sensor being configured to sense a first distance or velocity from a first position and in a first direction and output a corresponding first output signal,
- the second distance or movement sensor being configured to sense a first distance or velocity from a second position and in a second direction and output a corresponding second output signal,
- the third distance or movement being configured to sense sensing a first distance or velocity from a third position and in a third direction and output a corresponding third output signal,
∘ where, when projected on to a predetermined plane, no angle being 180° or more exists between adjacent directions,
- a controller configured to determine, from the first, second and third output signals, information relating to distances and/or relative movements between the sensor and the cavity along the first, second and third directions, respectively.

3. A system according to claim 1 or 2, wherein the controller is configured to, in the deriving step, determine that:
- a first distance from the housing to the inner surface along the first direction,
- a second distance from the housing to the inner surface along the second direction, and
- a third distance from the housing to the inner surface along the third direction,
all have varied within the predetermined period of time.

4. A system according to claim 3, wherein the controller is configured to, in the deriving step, derive information relating to a variation, within a predetermined period of time and in the plane, of an area encircled by the inner surface of the cavity.

5. A system according to any of the preceding claims, wherein the controller is configured to, in the deriving step, derive information relating to a deformation, in the plane, of the inner surface of the cavity.

6. A system according to any of the preceding claims, wherein the controller is configured to, in the deriving step, determine a relative movement, in the plane, of the housing in relation to the inner surface of the cavity.

7. A system according to any of the preceding claims, wherein the controller is configured to, in the deriving step, determine a period of the relative movements.

8. A system according to any the preceding claims, further comprising a resilient element and wherein at least one of the first, second and third distance or movement sensor is configured to output an output signal relating to a distance, along the pertaining direction, between the pertaining sensor and the resilient element.

9. A system according to claim 8, wherein the resilient element is configured to engage the inner surface of the cavity.

10. A system according to any of the preceding claims, wherein at least one of the first, a second and a third distance or movement sensors comprises Doppler-based sensor.

11. A system according to claim 10, wherein the Doppler-based sensor comprises a VCSEL with integrated Photodetector configured to launch radiation from the pertaining position and in the pertaining direction, receive radiation from the pertaining direction and output a pertaining output signal.

12. A system according to claim 10 or 11, wherein the at least one of the first, a second and a third distance or movement sensors further comprises a direction sensor configured to sense a direction of a relative movement between the inner surface and the pertaining sensor along the pertaining direction.

13. A system according to any the preceding claims, wherein at least one of the first, a second and a third distance or movement sensors comprises time-of-flight-based sensor.

14. A system according to claim 13, wherein the time-of-flight based sensor comprises a VCSEL with integrated Photodetector and a drive current supply configured to supply the VCSEL with integrated Photodetector with a varying drive current, the VCSEL with integrated Photodetector configured to launch radiation from the pertaining position and in the pertaining direction, receive radiation from the pertaining direction and output a pertaining output signal.

15. A method of deriving information relating to a cavity in blood perfused tissue comprising a cavity having an inner surface, the method comprising:
- providing a sensor in the cavity, the sensor comprising a housing and, in or at the housing, a first, a second and a third distance or movement sensor, the housing having an outer surface,
- the first distance or movement sensor sensing a first distance or velocity from a first position and in a first direction and outputting a corresponding first output signal,
- the second distance or movement sensor sensing a first distance or velocity from a second position and in a second direction and outputting a corresponding second output signal,
- the third distance or movement sensor sensing a first distance or velocity from a third position and in a third direction and outputting a corresponding third output signal, where, when projected on to a predetermined plane:
∘ a first tangent exists to the outer surface and intersecting the first direction,
∘ a second tangent exists to the outer surface and intersecting the second direction,
∘ a third tangent exists to the outer surface and intersecting the third direction,
∘ the first, second and third tangents define a closed figure inside which at least 50% of the housing exists, and
- determining, from the first, second and third output signals, information relating to distances and/or relative movements between the sensor and the cavity along the first, second and third directions, respectively.

16. A method of deriving information relating to a shape or a variation in the shape of a cavity in blood perfused tissue comprising a cavity having an inner surface, the method comprising:
- providing a sensor in the cavity, the sensor comprising a housing and, in or at the housing, a first, a second and a third distance or movement sensor, the housing having an outer surface,
- the first distance or movement sensor sensing a first distance or velocity from a first position and in a first direction and outputting a corresponding first output signal,
- the second distance or movement sensor sensing a first distance or velocity from a second position and in a second direction and outputting a corresponding second output signal,
- the third distance or movement sensor sensing a first distance or velocity from a third position and in a third direction and outputting a corresponding third output signal,
∘ where, when projected on to a predetermined plane, no angle being 180° or more exists between adjacent directions, and
- determining, from the first, second and third output signals, information relating to distances and/or relative movements between the sensor and the cavity along the first, second and third directions, respectively.
